## Europäisches Patentamt
## European Patent Office
## Office européen des brevets

(11) Publication number: **0 069 089**
**B1**

(12) # EUROPEAN PATENT SPECIFICATION

(45) Date of publication of patent specification: **15.10.86**

(51) Int. Cl.⁴: **A 61 F 2/14**

(21) Application number: **82830181.2**

(22) Date of filing: **17.06.82**

(54) Intraocular lens.

(30) Priority: **18.06.81 IT 3587281 u**

(43) Date of publication of application:
**05.01.83 Bulletin 83/01**

(45) Publication of the grant of the patent:
**15.10.86 Bulletin 86/42**

(84) Designated Contracting States:
**AT BE CH DE FR GB LI LU NL SE**

(56) References cited:
**FR-A-2 347 029**
**FR-A-2 469 918**
**FR-A-2 480 112**
**GB-A- 810 232**
**GB-A-2 053 689**
**US-A-4 041 552**
**US-A-4 268 921**
**US-A-4 327 450**

(73) Proprietor: **Surgivision Limited**
**Piermont House 33 Pier Road**
**St. Helier Jersey Channel Islands (GB)**

(72) Inventor: **Maggi, Carlo**
**Via Latina 8**
**I-00179 Roma (IT)**

(74) Representative: **Tonon, Gilberto et al**
**c/o Società Italiana Brevetti Piazza Poli 42**
**I-00187 Roma (IT)**

Courier Press, Leamington Spa, England.

## Description

This invention concerns a lens for the surgical correction of aphakia, occurring after removal of the crystalline lens from the eye, or refractive defects such as myopia, hypermetropia, or astigmatism.

The eyeball is a nearly spherical globe with outer walls of a fibrous, elastic consistency, for the most part opaque, with the exception of the area in front of the iris, which is transparent. The non-transparent part is named the sclera, while the transparent portion is the cornea.

Inside the eye, the space between the iris and the cornea is termed the anterior chamber, and is filled with a transparent aqueous liquid, the aqueous humour, whereas the remaining part is almost entirely filled with a transparent gel termed the vitreous body.

Behind the iris and in front of the vitreous body there is the posterior chamber consisting of the crystalline lens and the circular ciliary body which supports it and also is filled with aqueous humour.

After removal of the crystalline lens (such as in cataract operation) a high degree of hypermetropia occurs, which was formerly corrected by the use of spectacles or contact lenses. The best possible vision is however restored with the modern use of an intra-ocular lens surgically implanted, in either the anterior or posterior chamber. In the case of myopia or other defects of refraction, the implant must be inserted into the anterior chamber, in front of the still present crystalline lens.

All intra-ocular lenses, of which at the current state of ophthalmic technology there are many different versions, have a minimum diameter of 5—6 mm, whereas the previously mentioned anterior and posterior chambers of the eyeball have an internal diameter of approximately 10—11 mm, and 12—13 mm, respectively.

Consequently, in order for the lens to be placed in correspondence with the pupil, i.e. the circular aperture situated in the centre of the iris, it is necessary for it to have a supporting haptic portion, which in the various designs on the market has a variety of shapes, all more or less functional, generally including two or three flexible filiform loops, which are attached at one extremity to the lens and extend for a certain distance beyond the perimetral border of the latter, each one according to a pre-established direction.

In all previous designs, in order to ensure the support of the body of the lens in the above-mentioned position, the relative haptic portion is suspended between at least two diametrically opposite points of the basic perimeter of the anterior or posterior chamber in relation to the type of implant. When the lens is placed in the anterior chamber, the said haptic portion rests in the angle between the anterior surface of the iris and the cornea, while in the posterior chamber it rests in the angle between the posterior surface of the iris and the ciliary body, or, if it has a suitable shape, sits astride the pupillary aperture where it is lodged like a collar-stud, the haptic portion being responsible for the vertical equilibrium.

All previous types of intra-ocular lens have a total diametric length, including the optic and the haptic portions, not exceeding a total of 11—13 mm, i.e. the internal diameter of respectively the anterior and the posterior chambers.

A disadvantage not to be undervalued in the conventional type of intra-ocular lens is the difficulty of maintaining a stable position of the body of lens in exact correspondence with the pupil, due to the tendency of the supporting ocular tissues to undergo a process of atrophy due to the compression of the ocular tissues at the points of contact with the haptic portion of the lens, and this disadvantage has not been elimated by forseeing the possibility for certain types of lens to be sutured to the iris, due to the inconsistency and mobility of this organ.

Designs are also known (GB—A—810232, US—A—4327450 which was published after the claimed priority date of the present application) in which said haptic part is fixed into the eye wall, but such a fixing is carried out along a very short extent which does not ensure a firm attachment, and moreover the haptic part consists of rigid elements which therefore inevitably cause negative effects on the ocular tissues.

The aim of the present invention is to provide an intra-ocular lens which, although it comes under the general concept of previous similar lenses, is provided with an improved supporting haptic portion which consents the irremovable fixation of the body of the lens in correspondence with the pupillary aperture, without the risk even after an indefinite period of time of the slightest movement of the optic portion, for the impossibility of atrophy of the semi-rigid tissue of the scleral wall to which it is anchored, and for the large extension of the surface of contact between the tissues of the eye and the haptic portion of the lens, which minimises the weight of the implant.

The intra-ocular lens acording to the present invention is characterised by the fact that the supporting haptic portion comprises a plurality of support elements which are in the form of threads having no fixed form and are each of a length suitable for being held and anchored in an incision practised in the scleral wall of the eye during a surgical operation. Thus the minimum length of 19 mm of the present implant far exceeds the maximum length of any prior implant which has to be fitted like a spring into the anterior or posterior chamber. In fact the supporting haptic portions of the intra-ocular lens according to the present invention have a length of at least 7 mm, and are buried into the scleral wall through a suitable incision of the sclera, in whose thickness they run for at least 4.5 mm before perforating the said wall in correspondence with the base of the iris, thus penetrating into the anterior chamber and arriving at the lens near the pupillary aperture.

Thus the intra-ocular lens of the present invention constitutes a prosthesis with very different characteristics from those already existing, whose shape is dependent on the invariable anatomical features of the human eye. Even the surgical technique required to insert this new lens is completely different from any other ever used.

The present invention can be better illustrated by the following description, given by way of not limitative example, with reference to the enclosed drawing, in which:

figure 1 is a view of the intra-ocular lens in question seen from above;

figure 2 is a view similar to that in figure 1 of an embodiment of the intra-ocular lens in question;

figure 3 is a view similar to that in figures 1 and 2 of a further embodiment of intra-ocular lens;

figure 4 represents a further variation of embodiment; and

figure 5 is a diagrammatical view of the intra-ocular lens in question implanted in an aphakic eye.

With reference to the figures, a lens body is shown suitable for the surgical correction of aphakia by means of implantation in correspondence with the pupil in an eye which has undergone the removal of the crystalline lens.

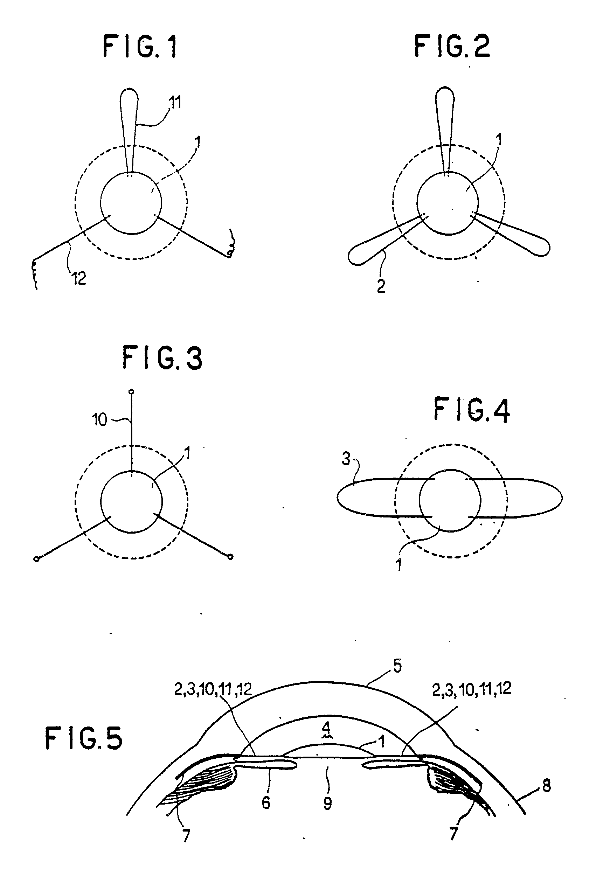

In figures 1 to 4 the dotted circle represents the average circumference of the posterior chamber of the eye, which has a diameter of 12—13 mm.

Figure 1 represents one form of general embodiment of the intra-ocular lens of the present invention. The lens body is indicated by 1 and on its perimeter the haptic parts 11, 12 are fixed at preferably constant angular intervals. The haptic parts are formed of threads, preferably having a section of 0.1 mm, and can take the form of loops or free threads. In the case of free threads these may have a total length of 5—6 cm, so that the surgeon may lay a suitable length in an incision conveniently prepared in the sclera, using the remaining portion of the thread as an anchoring suture in the sclera. Said threads may be of plastic, metal or any other suitable material of appropriate thickness and elasticity, and can also conveniently terminate in either atraumatic needles or a small final loop. Said threads when made of plastic material such as polypropylene once cut during surgery to the chosen length can have the end heated (for example by cautery) and so enlarged to improve anchorage in the tissues. A frontal view of this kind of anchorage is shown in figure 3. The threads constituting the haptic parts must be made of a material which is both inert and stable with regard to the ocular tissues, as is necessary in any surgical procedure.

The choice between the use of loops or free threads depends on the individual case of the patient undergoing operation, the fact remaining that in either case the haptic parts are buried in the scleral wall.

The present invention has a further advantage in the possibility of choosing the shape of the intra-ocular lens between various alternatives, a possibility which does not exist with the previous techniques of intra-ocular lenses, which latter are strictly limited by the anatomical characteristics of the inside of the eye. Therefore the total length of implants used up to the present time must slightly exceed the internal diameter of the anterior or the posterior chamber where they are fitted in a spring-like way. Thus it can be seen in figures 1 to 4 that while the haptic parts relative to previously used intra-ocular lenses extend at the most up to the dotted circumference, the loops and threads of the intra-ocular lens of the present invention present a notable lengthening of the said haptic parts beyond the dotted circumference, and said parts becoming integrated with the strong scleral tissues assume a supporting function in holding and maintaining the intra-ocular lens itself.

In figure 2 an intra-ocular lens is shown which presents three loops 2 disposed at 120° from each other fixed to the lens body 1.

In the embodiment shown in figure 3, the haptic part is formed of three threads 10 fixed to the lens 1 and which present at the free extremity an enlargement which serves to anchor it into the thickness of the scleral wall.

Said enlargement can be produced at the moment of application by means of heating the extremity of the thread after it has been cut to the desired length by the operator for that individual patient.

Alternatively, in order to anchor the threads to the tissues of the scleral wall, each of said threads may be terminated by an atraumatic needle or with a small loop.

A further example is shown in figure 4 in which the haptic part is formed by two loops 3 disposed at 180° from each other.

Although in their essential conformation the loops do not differ from conventional ones, their peculiarity with respect to the latter consists in the increased length which allows a substantial revolutionising of the method of surgical application of the intra-ocular lens in the present invention in comparison with that of similar known lenses.

The total length of the haptic part of the intra-ocular lenses produced up to the present, in addition to the diameter of the lens body, may not exceed the maximum diameter of the section of the eyeball in correspondence with the anterior or posterior chamber due to the methods of implant in use up to the present time, as previously mentioned.

As may be observed in figure 5, in the surgical application of the intra-ocular lens in question in an aphakic eye (i.e. without the crystalline lens) while the first stretch of the loops or of the threads, precisely that part as far as the dotted circumference in figures 1 to 4, corresponding to the length of conventional loops, is extended, like the latter, through the aqueous humour for the entire width of the anterior chamber 4 of the eye limited by the cornea 5 and the iris 6, the lengthening of each loop or thread provided by the present invention is placed in a respective incision 7 practised in the scleral wall 8 which is the only non-soft tissue of the eye, presenting a hard-elastic consistency so as to permit a firm anchoring of the loops or threads, with the elimination of any tendency to displacement of the lens body 1 from its correct position in exact correspondence with the pupil 9, as instead can occur with similar known lenses.

It is a free choice of the surgeon to extend the haptic parts of the lens as far as desired posteriorly to the said dotted circumference, but a minimum of 3—3.5 mm is recommended.

In this way the total length of the implant will be at least 19 mm, as compared with 13 mm of the largest implant previously available.

The present invention is not limited to the examples of embodiment described, but includes any variation of execution.

## Claims

1. An intraocular lens comprising a lens body (1) and a supporting haptic part, said haptic part including a plurality of support elements (2, 3, 10, 11, 12) attached to the edge of said lens body, said lens being characterised in that said support elements are in the form of threads having no fixed form and are each of an extent of at least 7 mm so as to be able to pass from the interior of the eye to the exterior of the eye and thereafter to be embedded in a surgically-made non-perforating incision located on the outer surface of the scleral wall and extending rearwards from the cornea.

2. Intraocular lens according to claim 1, in which said threads are in the form of free threads (10, 12) symmetrically or asymmetrically spaced from each other.

3. Intraocular lens according to claim 2, in which said threads are at least five centimeters long.

4. Intraocular lens according to claim 1, in which said threads are in the form of loops (2, 3) symmetrically or asymmetrically spaced from each other.

5. Intraocular lens according to claim 1, in which each of said threads presents at the free extremity a conformation permitting anchorage to the scleral wall.

6. Intraocular lens according to claim 5, in which each of said threads presents an enlargement of the free extremity.

7. Intraocular lens according to claim 5, in which each of said threads presents a small loop formed at the free extremity.

8. Intraocular lens according to claim 5, in which each of said threads presents an atraumatic needle applied to the free extremity.

9. Intraocular lens according to claim 2, in which said supporting haptic part of the lens body is formed of three free threads (10) disposed at 120° from each other.

10. Intraocular lens according to claim 4, in which said supporting haptic part of the lens body is formed of three threads in the form of loops (2) disposed at 120° from each other.

11. Intraocular lens according to claim 4, in which said supporting haptic part of the lens body is formed of two threads in the form of loops (3) disposed at 180° from each other.

12. Intraocular lens according to claim 1, in which said supporting haptic part of the lens body is formed of one thread (11) in the form of a loop and two free threads (12), disposed at 120° from each other.

## Patentansprüche

1. Intraokulare Linse, die einen Linsenkörper (1) und einen haptischen Trägerteil umfasst, wobei der haptische Teil mehrere an dem Rand des Linsenkörpers festgelegte Trägerelemente (2, 3, 10, 11, 12) umfasst, wobei die Linse dadurch gekennzeichnet ist, dass die Trägerelemente eine Gestalt von Faden, die keine starre Form besitzen, haben und eine Länge von mindestens 7 mm, sodass die Trägerelemente von dem Inneren des Auges bis zu dem Äusseren des Auges gehen können und danach in einem chirurgisch ausgeführten, nicht durchschlagenden Einschnitt, der sich auf der äusseren Oberfläche der skleralen Wand befindet und sich rückwärts von der Kornea ausdehnt, eingebettet werden können.

2. Intraokulare Linse gemäss Anspruch 1, worin die Fäden die Form von freien Fäden (10, 12) haben, die symmetrisch oder asymmetrisch im Abstand angeordnet sind.

3. Intraokulare Linse gemäss Anspruch 2, worin die Fäden mindestens 5 cm lang sind.

4. Intraokulare Linse gemäss Anspruch 1, worin die Fäden die Form von symmetrisch oder asymmetrisch im Abstand angeordneten Schleifen haben.

5. Intraokulare Linse gemäss Anspruch 1, worin jeder von den Fäden an dem freien Ende eine Gestaltung aufweist, die die Befestigung an der skleralen Wand ermöglicht.

6. Intraokulare Linse gemäss Anspruch 5, worin jeder von den Fäden eine Erweiterung des freien Endes aufweist.

7. Intraokulare Linse gemäss Anspruch 6, worin jeder von den Fäden eine kleine an dem freien Ende geformte Schleife aufweist.

8. Intraokulare Linse gemäss Anspruch 5, worin jeder von den Fäden eine untraumatische an dem freien Ende befestigte Nadel aufweist.

9. Intraokulare Linse gemäss Anspruch 2, worin der haptische Trägerteil des Linsenkörpers aus drei in einem Abstand von 120° angeordneten freien Fäden (10) besteht.

10. Intraokulare Linse gemäss Anspruch 4, worin der haptische Trägerteil des Linsenkörpers aus drei in einem Abstand von 120° angeordneten Fäden, die die Form von Schleifen (2) haben, besteht.

11. Intraokulare Linse gemäss Anspruch 4, worin der haptische Trägerteil des Linsenkörpers aus zwei in einem Abstand von 180° angeordneten Fäden, die die Form von Schleifen (3) haben, besteht.

12. Intraokulare Linse gemäss Anspruch 1, worin der haptische Trägerteil des Linsenkörpers aus einem schleifenförmigen Faden (11) und zwei freien Fäden (12), die in einem Abstand von 120° angeordnet sind, besteht.

**Revendications**

1. Lentille intraoculaire comprenant un corps de lentille (1) et une partie haptique de support, la dite partie haptique incluant une pluralité d'éléments de support (2, 3, 10, 11, 12) attachés au bord du dit corps de lentille, la dite lentille étant caractérisée en ce que les dits éléments de support ont la forme de fils n'ayant pas de forme fixe et ont chacun une longueur d'au moins 7 mm de façon à pouvoir passer de l'intérieur de l'oeil à l'extérieur de l'oeil et succéssivement à être logés dans une incision chirurgicale non perforante située sur la surface extérieure de la paroi sclérale et s'étendant à l'arrière de la cornée.

2. Lentille intraoculaire selon la revendication 1, dans laquelle les dite fils ont la forme de fils libres (10, 12) espacés symétriquement ou asymétriquement l'un de l'autre.

3. Lentille intraoculaire selon la revendication 2, dans laquelle les dits fils ont une longueur d'au moins 5 cm.

4. Lentille intraoculaire selon la revendication 1, dans laquelle les dits fils ont la forme d'anses (2, 3) symétriquement ou asymétriquement espacés l'un de l'autre.

5. Lentille intraoculaire selon la revendication 1, dans laquelle chacun des dits fils présente à l'extrémité libre une conformation permettant l'ancrage à la paroi sclérale.

6. Lentille intraoculaire selon la revendication 5, dans laquelle chacun des dits fils présente un regonflement de l'extrémité libre.

7. Lentille intraoculaire selon la revendication 5, dans laquelle chacun des dits fils présente une petite anse formée à l'extrémité libre.

8. Lentille intraoculaire selon la revendication 5, dans laquelle chacun des dits fils présente une aiguille atraumatique appliquée à l'extrémité libre.

9. Lentille intraoculaire selon la revendication 2, dans laquelle la dite partie haptique de support du corps de lentille est formée de trois fils libres (10) disposés à 120° l'un de l'autre.

10. Lentille intraoculaire selon la revendication 4, dans laquelle la dite partie haptique de support du corps de lentille est formée de trois fils ayant la forme d'anses (2) disposés à 120° l'un de l'autre.

11. Lentille intraoculaire selon la revendication 4, dans laquelle la dite partie haptique de support du corps de lentille est formée de deux fils ayant la forme d'anses (3) disposés à 180° l'un de l'autre.

12. Lentille intraoculaire selon la revendication 1, dans laquelle la dite partie haptique de support du corps de lentille est formée d'un fils (11) ayant la forme d'une anse et de deux fils libres (12) disposés à 120° l'un de l'autre.

0 069 089

# FIG.1

# FIG.2

# FIG.3

# FIG.4

# FIG.5

1